# EUROPÄISCHE PATENTSCHRIFT

(11) **EP 2 465 572 B1**
(45) Veröffentlichungstag und Bekanntmachung des Hinweises auf die Patenterteilung: **09.11.2016**
(21) Anmeldenummer: 11191738.1
(22) Anmeldetag: 02.12.2011
(51) Int. Cl.: A61N 1/05, A61N 1/08

(54) **MRT-kompatible implantierbare Leitung**
MRI-compatible implantable lead
Fil implantable compatible avec l'IRM

(30) Priorität: 17.12.2010 US 424073 P
(43) Veröffentlichungstag der Anmeldung: 20.06.2012
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Galander, Marcus

(56) Entgegenhaltungen:
- EP-A2- 2 359 898
- WO-A1-2008/115426
- US-A1- 2006 247 684
- US-A1- 2006 252 314
- US-A1- 2008 132 985
- US-A1- 2009 149 920
- US-A1- 2010 217 262

## Beschreibung

Die Erfindung betrifft ein permanent oder temporär implantierbares Gerät mit einem langgestreckten elektrischen Leiter.

Solche Geräte, beispielsweise Elektrodenleitungen für die Elektrostimulation oder Katheter für elektrophysiologische Interventionen, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Auch können solche induzierten Ströme über Elektrodenpole der Elektrodenleitung an umliegendes Gewebe abgegeben werden und so beispielsweise zu unerwünschten Gewebeerwärmungen führen. Deshalb können Herzschrittmacherpatienten heutzutage in der Regel nicht oder nur eingeschränkt in einem Kernspintomografen untersucht werden.

An implantierbaren Herzschrittmachern oder Defibrillatoren (im Folgenden auch gemeinsam als Herzstimulatoren oder IPG (implantable pulse generator) bezeichnet) sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

EP-A-2 359 898 (veröffentlicht nach dem Prioritätsdatum der vorliegenden Anmeldung) und US-A-2010/217262 offenbaren unterschiedliche Lösungen dieses Problems. Der Erfindung liegt die Aufgabe zugrunde, ein Gerät zu schaffen, welches das zuvor beschriebene Problem löst.

Erfindungsgemäß wird diese Aufgabe durch ein temporär oder permanent implantierbares medizinisches Gerät gemäß Anspruchs 1 gelöst.

Es sei bereits hier darauf hingewiesen, dass im Folgenden die Begriffe Funktionsleiter und therapieführender Leiter synonym verwendet werden.

Der Wellenwiderstand ist ein Maß für den wellenlängen-abhängigen Wert des elektrischen Widerstands von HF-Kabeln.

Bei Koaxialkabeln ist der Wellenwiderstand unabhängig von der Kabellänge aber abhängig vom Kapazitäts- und Induktivitätsbelag des HF-Kabels. Diese Werte sind direkt abhängig vom Durchmesser des Innenleiters und der Schirmung und von der Dielektrizitätskonstanten des Dielektrikums. Kapazitäts- und Induktivitätsbelag eines HF-Kabels können im Ersatzschaltbild durch eine Reihenschaltung von vielen einzelnen Induktivitäten und eine Parallelschaltung ebenso vieler Kapazitäten nachgebildet werden. Unter Vernachlässigung des ohmschen Widerstands ergibt sich der Wellenwiderstand (Z) näherungsweise aus der Wurzel des Verhältnisses von Induktivität zur Kapazität.

Die Erfindung schließt den Gedanken ein, den Wellenwiderstand eines Funktionsleiters so einzustellen, dass der Funktionsleiter für Frequenzbereiche, die denjenigen zu erwartender Störfelder entsprechen, einen hohen Wellenwiderstand besitzt und Ströme dieser Frequenz entsprechend dämpft.

In Fortführung des Erfindungsgedankens wird dies durch eine entsprechend ausgelegte Mantelwellensperre erreicht und zwar indem der eine Doppelleitung bildende Abschnitt des medizinischen Gerätes als Mantelwellensperre ausgebildet ist.

Das medizinische Gerät ist vorzugsweise eine Elektrodenleitung zum Anschluss an einen implantierbaren Herzstimulator.

Alternativ ist das medizinische Gerät ein Katheter für elektrophysiologische Interventionen oder eine Elektrodenleitung für einen temporären Herzstimulator.

Vorzugsweise ist die Koppelimpedanz zwischen Funktionsleiter und Zusatzleiter von einer Kapazität gebildet. Alternativ oder zusätzlich kann die Koppelimpedanz auch von einer Induktivität und/oder einem Kurzschluss gebildet sein.

Gemäß alternativer vorteilhafter Ausgestaltungen kann der als Doppelleitung ausgebildete Abschnitt des medizinischen Gerätes als Koaxial-, als Parallelleitung, Stripline oder dergleichen aufgebaut sein.

Besonders bevorzugt ist eine Ausführungsvariante der Erfindung bei der der als Doppelleitung ausgebildete Abschnitt des medizinischen Geräts in dieses konstruktiv so integriert ist, dass die geometrische Strecke zwischen Funktionsleiter und Zusatzleiter, die von der Koppelimpedanz (z.B. einem Kondensator) überbrückt wird, kurz ist im Vergleich zur Wellenlänge in der Doppelleitung für eine vorgegebene höchste Arbeitsfrequenz. Das Verhältnis geometrische Strecke zwischen Funktionsleiter und Zusatzleiter zur Wellenlänge bei vorgesehener höchster Arbeitsfrequenz ist vorzugsweise kleiner als ein Zehntel (1/10).

Besonders bevorzugt ist eine Ausführungsvariante, bei der das medizinische Gerät ein- oder mehrpolarer, temporär anwendbarer Katheter oder eine dauerimplantierbare Elektrodenleitung oder ein langgestrecktes elektrisch leitfähiges Implantat mit einer teilweisen Isolation ist, so dass an definierten Elektrodenflächen eine lokale Erwärmung durch im MRT induzierte Ströme zu erwarten ist. Hierbei ist proximal eines Elektrodenpoles zumindest abschnittsweise um den oder nahe an dem Zuleitungsdraht (therapieführender Leiter, hier als Funktionsleiter bezeichnet) ein zweiter Leiter (hier als Zusatzleiter bezeichnet) angeordnet, der zusammen mit dem therapieführenden Leiter auf diesem Abschnitt eine Doppelleitung bildet, wobei die Leiter durch ein Dielektrikum (230) voneinander isoliert sind. Die Enden des zweiten Leiters, also des Zusatzleiters, sind über eine Impedanz miteinander verbunden, deren Wert in Abhängigkeit der Induktivitäts- und Kapazitätsbeläge des Doppelleitungsabschnitts so bestimmt ist, dass die therapeutische Zuleitung für Frequenzen wesentlich höher als die von Therapie- und Diagnosesignalen eine hohe Impedanz aufweist (bzw. hier Ströme stark dämpft) und zwar für zumindest eine MR typische HF Frequenz mit dem Ziel, die unerwünschte Implantats/Elektrodenerwärmung während der MR Bildgebung/Spektroskopie zu reduzieren/vermeiden.

Vorzugsweise ist der Doppelleitungsabschnitt ist so realisiert, dass der eine Leiter der therapieführende Funktionsleiter selbst ist, der zur Realisierung der Mantelwellensperre nicht unterbrochen ist, indem die Mantelwellensperre um den Funktionsleiter herum oder diesem unmittelbar benachbart angeordnet ist. Es ist dann keine potentiell unzuverlässige Kontaktierungstechnik am therapieführenden Leiter (Funktionsleiter) selbst erforderlich.

Vorzugsweise ist der Doppelleitungsabschnitt gewendelt, d.h. helixförmig konstruiert.
besonders bevorzugt ist eine Ausführungsvariante, bei der der Doppelleitungsabschnitt eine Schleife bildet, die parallel zu dem Funktionsleiter oder den Funktionsleitern, also den therapieführenden Leitern, in der Elektrodenzuleitung geführt ist oder bei gewendelten E-lektrodenleitungskonstruktionen in die Wendellücken mitgewendelt ist.

Im Falle von Seilelektroden, bei denen der therapieführende Funktionsleiter ein Seilleiter ist, ist es bevorzugt, wenn der Funktionsleiter wird im Doppelleitungsabschnitt (koaxialen Abschnitt) als Wendelstruktur ausgeführt ist, so dass Der Funktionsleiter ist in dem als Doppelleitung ausgebildeten Abschnitt des medizinischen Gerätes helixartig gewendelt ist.

Als Dielektrikum wird vorzugsweise die Isolation des therapieführenden Leiters, also des Funktionsleiters, selbst genutzt. Der zweite Leiter der Doppelleitung, also der Zusatzleiter, kann dann durch Metallisierung des Dielektrikums (z.B. durch Aufdampfen einer Metallschicht) realisiert und somit beispielsweise von einer aufgedampften Metallschicht gebildet sein. Aus dieser Weise ergibt sich ein ebenso einfacher wie kompakter Aufbau des Doppelleitungsbschnitts.

Alternativ kann der Zusatzleiter, z.B. der koaxiale Außenleiter aus einem flexiblen, leitfähigen Polymer oder Liquid Cristal Polymer (LCP) bestehen.

Auch kann der Doppelleitungsabschnitt als getrennt gefertigtes Bauelement in die Elektrodenleitung eingebaut sein (mit herkömmlicher Verbindungstechnik).

Vorzugweise hat der Doppelleitungsabschnitt einen Wellenwiderstand von weniger als 120 Ohm.

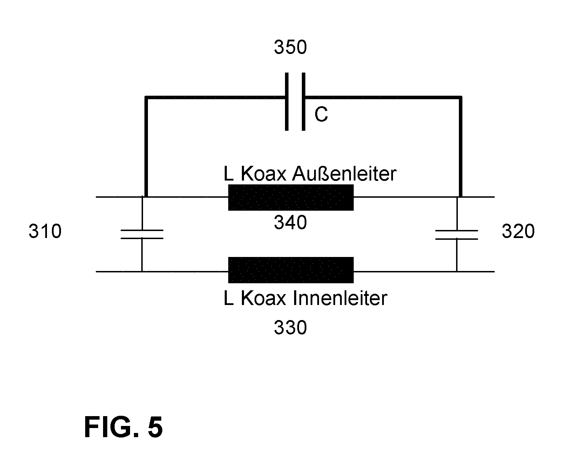

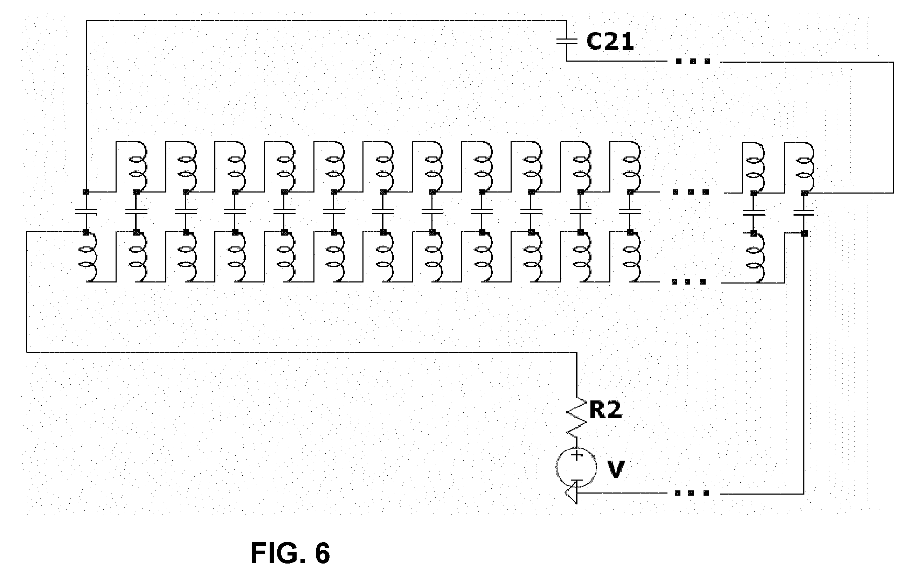

Der Doppelleitungsabschnitt kann auch durch diskrete Bauelemente realisiert sein, und zwar vorzugsweise mit weniger als 20 Teilabschnitten (d.h. "La-Cc-Lb-Zellen") so wie es sich aus dem Ersatzschaltbildern in Figuren 5 und 6 ergibt.

Der Doppelleitungsabschnitt kann auch (hybrid) als Leitung aber mit längs der Leitung ein oder mehrfach unterbrochenem Dielektrikum ausgeführt sein, so dass die Kondensatoren Cc diskret realisiert sind.

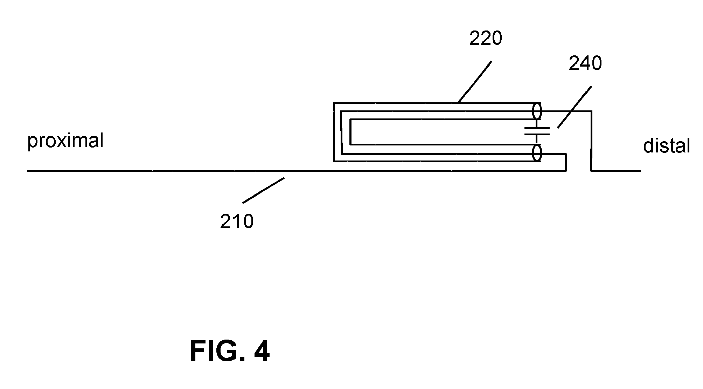

Der Doppelleitungsabschnitt ist vorzugsweise so aufgebaut, dass die Verluste (Längswiderstände der Leiter, d.h. die der Induktivitäten La und Lb oder Parallelwiderstände der Kapazitäten Cc, siehe Abb4) so abgestimmt sind, dass die Bandbreite um die Resonanzfrequenz(en) weniger als 10 MHz betragen.

Die erfindungsgemäße Mantelwellensperre ist vorzugsweise so aufgebaut, dass die durch sie hervorgerufene Dämpfung des (wärmeerzeugenden) Stroms für die jeweilige Larmorfrequenz der MR Bildgebung oder Spektroskopie größer als 6 dB ist.

Vorzugsweise liegt das Verhältnis der Induktivitätsbeläge der einzelnen Leiter der Doppelleitung (entspricht dem Verhältnis La/Lb in Figur 6) zwischen 0.1 und 10, und ist bevorzugt Lb/La = 0,75.

Der Doppelleitungsabschnitt ist vorzugsweise so aufgebaut, dass durch die Parameter der vom Doppelleitungsabschnitt gebildeten Mantelwellensperre die Frequenzabstände der Resonanzen so optimiert sind, dass möglichst viele HF Arbeitsfrequenzen von MRT Geräten mit derselben Mantelwellensperre (und damit der selben Elektrodenleitung) getroffen werden. Bevorzugt liegen die Resonanzen möglichst eng beieinander, mit einem Frequenzabstand von weniger als 25 MHz.

Die Erfindung soll nun anhand von Ausführungsbeispielen mit Bezug auf die Figuren näher erläutert werden. Die Figuren zeigen Folgendes:
- Fig. 1: zeigt als implantierbare medizinische Geräte einen implantierbaren Herzstimulator 10 und eine daran angeschlossene implantierbare Elektrodenleitung 20.
- Fig. 2: zeigt beispielhaft einen Temperaturverlauf an der Elektrodenspitze unter Einfluss von hochfrequenten Wechselfeldern, wie sie in einem Kernspintomografen (MRT) herrschen.
- Fig. 3: zeigt den konstruktiven Aufbau einer ferritfreien Mantelwellensperre.
- Fig. 4: zeigt einen bevorzugten Einbau einer Mantelwellensperre in eine Elektrodenleitung.
- Fig. 5: zeigt ein vereinfachtes Ersatzschaltbild der erfindungsgemäßen Realisierung einer Mantelwellensperre.
- Fig. 6: zeigt ein detaillierteres Ersatzschaltbild (Spice-Modell), das die Doppelleitung diskretisiert als ein LC Netzwerk darstellt.
- Fig. 7A und 7B: zeigen das Verhalten zweier Elektrodenleitungen mit unterschiedlich ausgeführter Mantelwellensperre.
- Fig. 8A und 8B: zeigen eine als Wendel konstruierte Realisierungsform einer Mantelwellensperre in Koaxialtechnik.
- Figuren 9A bis 9C: zeigen verschiedene Ausführungsbeispiele einer Doppelleitung.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten-Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

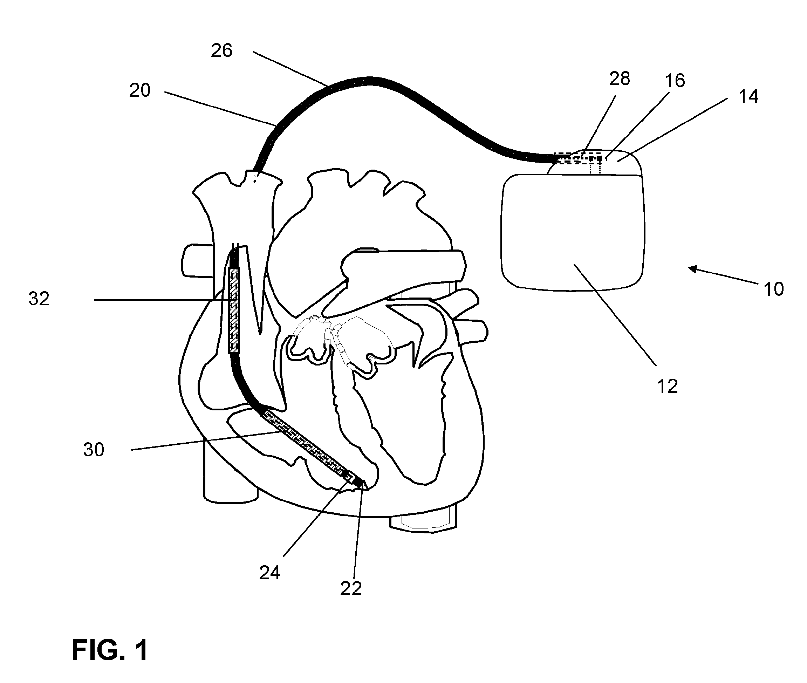

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren. Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

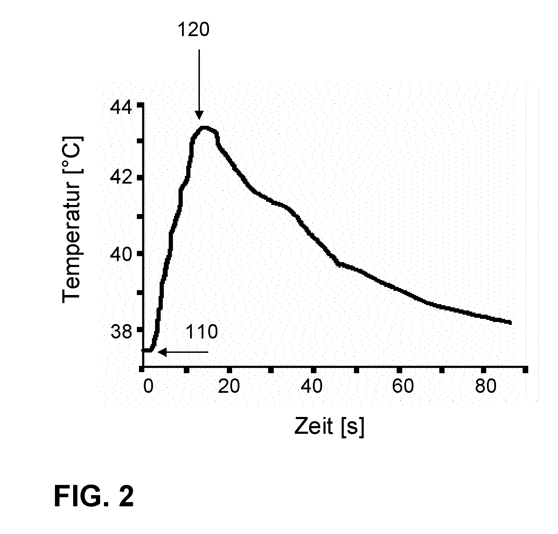

In Figur 2 ist ein typischer Temperaturverlauf 100 einer konventionellen Schrittmacher/ICD-Elektrode im Magnetresonanztomografen (MRT) dargestellt. Mit dem Einschalten des hochfrequenten Wechselfeldes im Tomografen zum Zeitpunkt 110 steigt die Temperatur rasch an, wobei die Steilheit des Anstieges und die maximal erreichbare Temperatur stark von der Elektrodenlage bezogen auf die hochfrequenten Wechselfelder des MRT abhängig ist. Wird das hochfrequente Wechselfeld abgeschaltet (zum Zeitpunkt 120), dann kühlt sich die Elektrodenspitze aufgrund ihrer vergleichsweise geringen Wärmekapazität verhältnismäßig schnell wieder ab.

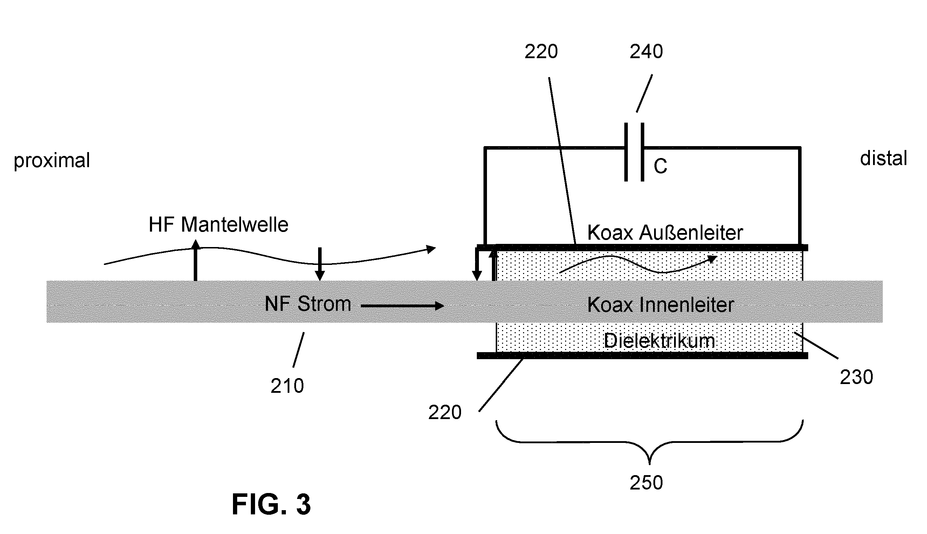

In Figur 3 ist die konstruktive Ausführung der ferritfreien Mantelwellensperre dargestellt. In Figur 3 ist der Leiter 210 der Einfachheit halber gestreckt gezeichnet, im Allgemeinen können die Leiter auch gewendelt sein. Der Leitungsabschnitt im Bereich des Bezugszeichens 250 bildet einen Doppelleitungsabschnitt, in dem ein Dielektrikum 230 die Leiter, nämlich den Funktionsleiter 210 und den Zusatzleiter 220 voneinander trennt. Der Leitungsabschnitt 250 - im Folgenden auch Doppelleitung genannt - kann als Koaxialleiter, Parallelleiter, Stripline oder dergleichen realisiert sein, wobei im Folgenden ohne Ausschluss der Allgemeinheit die Ausführungen mit Koaxialleiter beschrieben werden. In dem abgebildeten Ausführungsbeispiel bildet der Funktionsleiter 210 innerhalb des Doppelleitungsabschnitts 250 einen Koax-Innenleiter und der Zusatzleiter ist der Koax-Außenleiter 220.

Gemäß einer bevorzugten Realisierung ist dieser Leiterabschnitt gewendelt. Die Realisierung ist so, dass die geometrische Strecke, die der Kondensator 240 überbrückt, kurz ist im Vergleich zur Wellenlänge in der Doppelleitung für die höchste Arbeitsfrequenz (bevorzugt Faktor <1/10). Ein Merkmal dieser Erfindung ist es, dass so eine Wendel auch im Falle von Seil-Elektroden realisiert werden kann, um diesen kurzen Abstand zu realisieren. Eine andere bevorzugte Variante ist in Figur 4 gezeigt.

Die Kapazität C 240 koppelt das am distalen Ende des koaxialen Außenleiters 220 herauskommende Feld auf kurzem elektrischem Weg phasenverkehrt an den proximalen Eingang in den koaxialen Abschnitt 220 zurück und unterdrückt somit die Mantelwelle. Die hochfrequenten Signale werden wegen des Skineffekts an der Oberfläche geleitet bzw. die Felder pflanzen sich gar durch die umliegende Isolation (Verschiebungsströme) fort. Man spricht daher von Mantelwellen. Die niederfrequenten Therapieströme fließen hingegen im Inneren des Leiters. Mit der erfindungsgemäßen Sperre sollen nur die Mantelwellen geblockt werden, die also die unerwünschte HF Energie transportieren.

Figur 4 zeigt einen bevorzugten Einbau einer Mantelwellensperre in eine Elektrodenleitung. Die Mantelwellensperre realisiert aus 220 und 240 (dazwischen das Dielektrikum - hier nicht gezeigt) ist bevorzugt nahe des distalen Endes der Elektrodenleitung montiert, bevorzugt in der distal-seitigen Hälfte der Elektrodenleitung

Kennzeichnend für die Erfindung ist, dass der eine Leiter der Doppelleitung durch den therapeutischen Leiter (den Funktionsleiter) der Elektrodenzuleitung selbst realisiert ist, Dielektrikum und Zweitleiter (Zusatzleiter) nur nebenan oder rundherum geführt sind, d.h. ohne das der therapeutische Leiter mechanisch unterbrochen werden muss. Dies ist ein entscheidendes Zuverlässigkeitsmerkmal der erfindungsgemäßen Lösung. Der Kondensator verbindet die Enden des Zusatzleiters. Die Kontaktierung hier ist nur für die MR-Eigenschaften der Elektrode relevant nicht aber für ihre lebens-lange therapeutische Zuverlässigkeit.

Die Verschaltung der so konstruierten Doppelleitung mit dem Kondensator 240 in der beschriebenen Weise realisiert eine Mantelwellensperre. Gemäß der Erfindung ist diese so realisiert, dass sie bei mindestens einer Frequenz resonant ist. Bei dieser Resonanzfrequenz bzw. bei den mehreren Resonanzfrequenzen ist die Wirkung maximal. Erfindungsgemäß sind diese Resonanzfrequenzen so gelegt, dass sie nahe der Arbeitsfrequenz gängiger MRT Geräte liegen (siehe Tabelle 1) und zwar so, dass die Bandbreiten dieser Resonanzen max. 10 MHz betragen.

Nach dem Stand der Technik werden als Mantelwellensperren für den Frequenzbereich üblicher MRT-Geräte Ferrite eingesetzt. Diese sättigen jedoch im statischen Feld des MRT und werden damit unwirksam. Daher ist es Ziel dieser Erfindung eine Lösung ohne Ferrite auszuarbeiten.

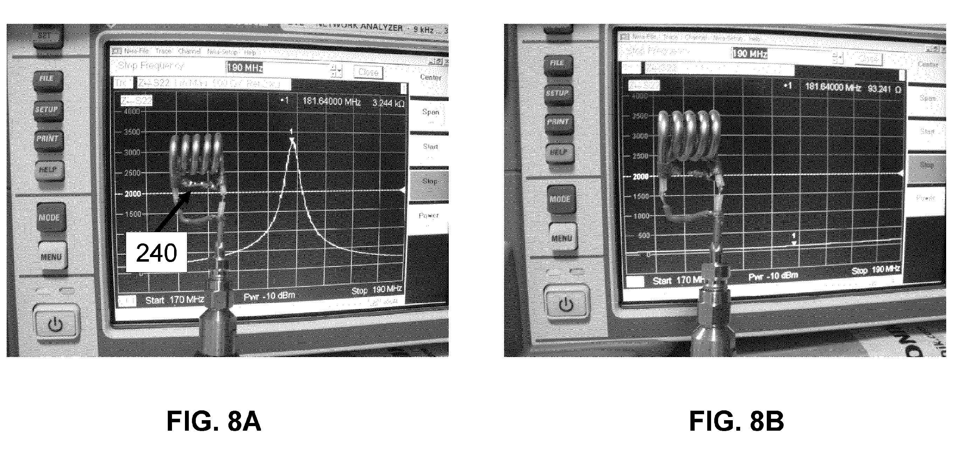

Eine beschriebene Implementierung ist die gewendelte Ausführung. In der Regel reichen wenige Wicklungen aus, dann braucht auch die Kapazität C 240 keine langen Zuleitungen, die sonst eine unerwünschte parasitäre Induktivität hätten. Eine Konstruktionsvariante ist beispielhaft in Figur 8 dargestellt.

In Figur 5 ist das Ersatzschaltbild der erfindungsgemäßen ferritfreien Mantelwellensperre dargestellt, die bei dem Ausführungsbeispiel in Figur 3 als koaxialer Leitungsabschnitt ausgebildet ist. In dem Ersatzschaltbild sind der Innenleiter durch die Induktivität 330 und Außenleiter durch die Induktivität 340 dargestellt, das Dielektrikum über die Koppelkondensatoren 310, 320. Die zusätzliche Kapazität 350 bewirkt eine Phasenverschiebung und koppelt so die Mantelwelle destruktiv zurück. Wichtig ist zu erwähnen, dass sich zwar der koaxiale Innenleiter im Ersatzschaltbild als eine Induktivität 330 dargestellt, konstruktiv aber nicht durch ein zusätzliches Bauelement unterbrochen ist oder die gezeigten Koppelkapazitäten 320 mit diesem therapieführenden Funktionsleiter verbunden sind.

Figur 6 zeigt ein detaillierteres Ersatzschaltbild in Form eines Splice-Modells, das die Doppelleitung diskretisiert als ein LC Netzwerk darstellt. Berücksichtigt ist auch, dass die Elemente real verlustbehaftet sind (nicht dargestellt, aber im Spice Modell berücksichtigt). Die in den vorigen Figuren mit 240 oder 350 bezeichnete Kapazität heißt hier C21. Der Widerstand und die Spannungsquelle gehören zur Messschaltung die die Funktionsweise erläutert. Der Strom der durch den Messwidersand R2 fließt würde im Falle einer realen Elektrode in das Gewebe fließen und es entsprechend während MR Bildgebung erwärmen.

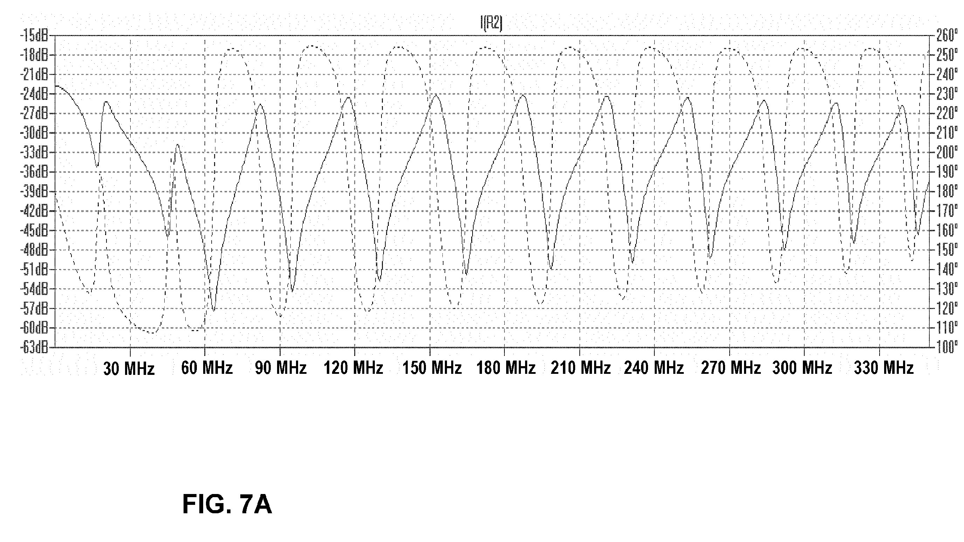

Figur 7A zeigt das Ergebnis einer Simulation auf Basis des Splice-Modells aus Figur 6 für eine multiresonante Mantelwellensperre nach erfindungsgemäßen Prinzip, die dafür optimiert ist, dass dieselbe Elektrode bei allen 3 in Tabelle 1 angegebenen MRT Geräten wirksam geschützt ist. Die Wirkung ist bei den Frequenzen besonders ausgeprägt, wo der Strom stark gedämpft wird. Gezeigt sind Amplituden und Phasenlage des Stroms im Messwiderstand.

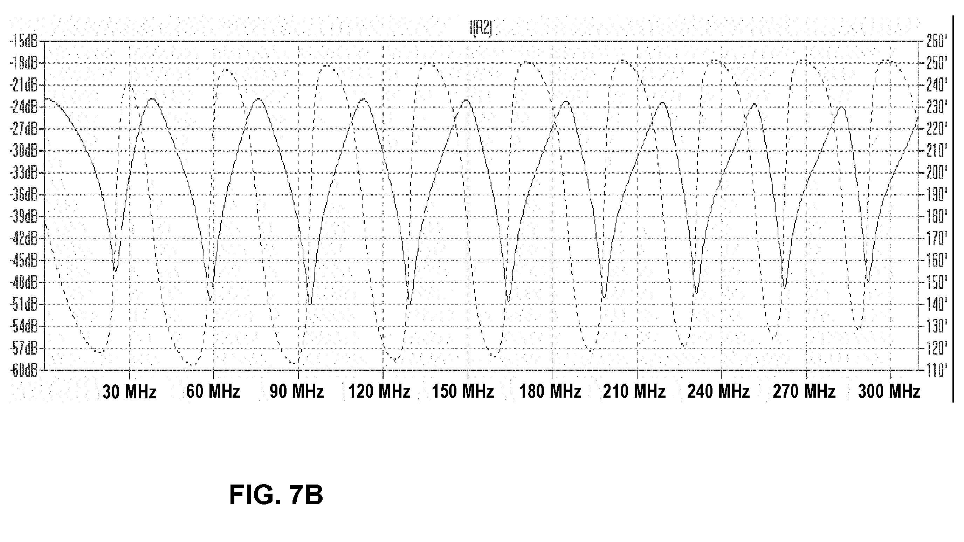

Figur 7B zeigt das Ergebnis einer ähnlichen Simulation, bei der die modellierte Mantelwellensperre statt des Kondensators 240. bzw. C21 eine Induktivität verwendet. Hier wird nur phänomenologisch die ähnliche Wirkung gezeigt. Über entsprechende Parameteranpassungen können auch auf diesem Wege die Wunschfrequenzen getroffen werden.

**Tabelle 1**

| Frequenz (MHz) / MR Magnetfeldstärke (T) | Dämpfung (dB) des Stroms bezogen auf ein Signal bei 1 kHz | Reduktion der Erwärmung um (%) |
|---|---|---|
| 63.5 MHz / 1.5 T | 34.5 dB | 99.9 % |
| 126.9 MHz / 3 T | 20.3 dB | 99.0 % |
| 296.1MHz/7T | 17 dB | 98.0 % |

Tabelle 1 zeigt Arbeitsfrequenzen bei einem gyromagnetischen Verhältnis von 42.3 MHz/T für Protonen. Die Wirkung der Figur 7A zugrunde liegenden Ausführungsvariante wird bezogen auf ein therapeutisch/diagnostisches Nutzsignal bei 1KHz angegeben, da bis zu dieser Frequenz aus therapeutischen und diagnostischen Gründen die Elektrode möglichst ungedämpft funktionieren muss

Die Resonanzen der erfindungsgemäßen Mantelwellensperre liegen physikalisch-bedingt in bestimmten Frequenzabstandsverhältnissen. Daher können die gewünschten Arbeitsfrequenzen von MR Geräten nicht alle gleichgut getroffen werden. Die Optimierung der in Abb4 dargestellten Komponenten (insbesondere auch hinsichtlich der hier gewollten Verluste zur Verbreiterung des Resonanztiefs) ermöglicht jedoch einen reichlich guten Kompromiss.

Eine diesbezüglich bevorzugte Realisierung ist wie folgt, wobei die Leitung in 40 Segmente diskretisiert ist. Die real zu verwendende Leitung muss so konstruiert sein, dass sie das gleiche verhalten aufweist wie dies Ersatzschaltbild mit folgenden Werten:
Leiterteilinduktivitäten
   La=7.4e-9 (H);
   Lb=0.75*La (H);
Mit folgenden seriellen Verlustwiderständen
   RLa=0.1 (Ohm);
   RLb=0.1 (Ohm);
Die Leitung soll einen Wellenwiderstand von
   Z0=18.75 (Ohm);
Haben, daraus errechnet sich die, Querkapazität pro Segment
   Cc=(La+Lb)/Z0^2 (F);
Mit einem Parallelen Verlustwiderstand von
   CcRp=1e6 (Ohm);
Der hier angenommene Quellenwiderstand (ist gleichzeitig der Messwiderstand) ist Rq=20 Ohm;

Eine als Wendel konstruierte Realisierungsform ist Figur 8 gezeigt. Die abgebildete Realisierung ist nur beispielhaft, daher ist hier die Resonanz nicht bei einer typischen MR Frequenz kann aber über einen entsprechenden Kondensator leicht darauf abgestimmt werden. Die in Figur 8 dargestellte Ausführungsform zeigt eine gewendelten Realisierung der Mantelwellensperre in Koaxialtechnik. auf dem dahinter abgebildeten Bildschirm ist die Impedanz aufgetragen und ein deutlicher Peak zu erkennen wenn der Kondensator 240 den Koax-Außenleiter an seinen Enden zusammenschließt (Figur 8a). Dies ist nicht der Fall wenn der Kondensator fehlt (Figur 8b).

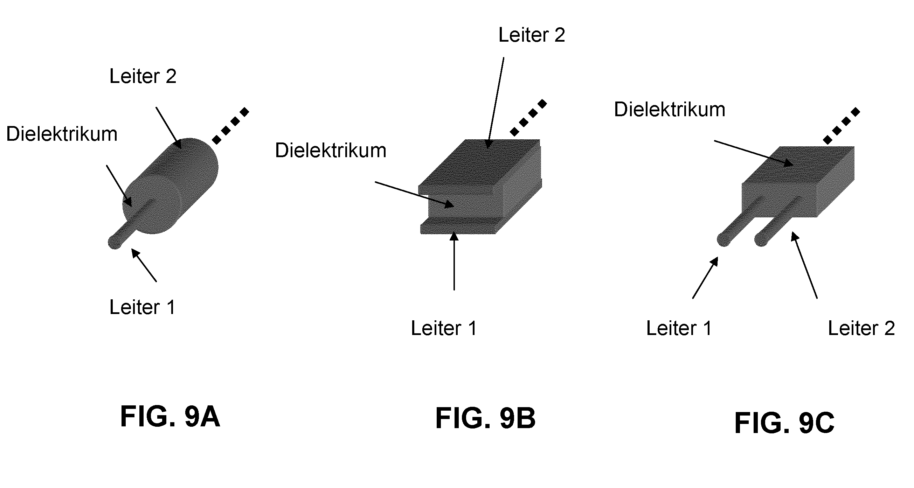

Figuren 9A bis 9C zeigen verschiedene Ausführungsbeispiele einer Doppelleitung. In Figur 9A ist der Doppelleitungsabschnitt als Koaxialleiter ausgebildet. In Figur 9B ist der Doppelleitungsabschnitt als Parallelleitung zweier Flachleiter ausgebildet. In Figur 9C ist der Doppelleitungsabschnitt als Parallelleitung zweier Stripleiter ausgebildet.

## Patentansprüche

1. Temporär oder permanent implantierbares medizinisches Gerät mit wenigstens einem langgestreckten elektrischen Funktionsleiter (210) für die Übertragung von Therapiesignalen oder Diagnosesignalen oder beidem,
wobei wenigstens ein Zusatzleiter (220) vorgesehen ist, der zusammen mit dem Funktionsleiter (210) wenigstens abschnittsweise eine Doppelleitung bildet, von dem Funktionsleiter (210) durch ein Dielektrikum (230) getrennt ist und der mit dem Funktionsleiter über eine Koppelimpedanz gekoppelt ist, wobei die Enden des Zusatzleiters (220) über eine Impedanz miteinander verbunden sind,
wobei die Impedanz dieser Verbindung so bemessen ist, dass der Wert des Leitungswellenwiderstands des Funktionsleiters (210) für Frequenzbereiche weit oberhalb eines Frequenzbereichs der Therapie- oder Diagnosesignale, also im Bereich von HF-Feldern, wesentlich größer ist als der Wert des Leitungswellenwiderstands des Funktionsleiters (210) im Frequenzbereich der Therapie- oder Diagnosesignale, so dass Ströme in einem oberhalb des Frequenzbereichs der Therapie- oder Diagnosesignale mindestens 6 dB stärker gedämpft werden als Ströme im 1kHz Bereich, wobei der eine Doppelleitung bildende Abschnitt des medizinischen Gerätes eine Mantelwellensperre bildet, und wobei die Koppelimpedanz von einer Kapazität oder einer Induktivität gebildet wird.

2. Medizinisches Gerät nach Anspruch 1, **dadurch gekennzeichnet, dass** das medizinische Gerät eine Elektrodenleitung (20) zum Anschluss an einen implantierbaren Herzstimulator (10) ist.

3. Medizinisches Gerät nach einem der Ansprüche 1 oder 2, **dadurch gekennzeichnet, dass** die Koppelimpedanz von einer Kapazität (240, 350) gebildet ist.

4. Medizinisches Gerät nach einem der Ansprüche 1 bis 3, **dadurch gekennzeichnet, dass** die Koppelimpedanz von einer Induktivität gebildet ist.

5. Medizinisches Gerät nach einem der Ansprüche 1 bis 4, **dadurch gekennzeichnet, dass** der als Doppelleitung ausgebildete Abschnitt des medizinischen Gerätes als Koaxial-, als Parallelleitung oder Stripline aufgebaut ist.

6. Medizinisches Gerät nach einem der Ansprüche 1 bis 5, **dadurch gekennzeichnet, dass** der als Doppelleitung ausgebildete Abschnitt des medizinischen Geräts in dieses konstruktiv so integriert ist, dass die geometrische Strecke zwischen Funktionsleiter (210) und Zusatzleiter (220), die von der Koppelimpedanz überbrückt wird, kurz ist im Vergleich zur Wellenlänge in der Doppelleitung für eine vorgegebene höchste Arbeitsfrequenz.

7. Medizinisches Gerät nach einem der Ansprüche 1 bis 6, **dadurch gekennzeichnet, dass** der Zusatzleiter (220) proximal eines Elektrodenpols (22, 24, 30, 32) angeordnet ist und dem Funktionsleiter (210) zumindest abschnittsweise nahe benachbart ist oder diesen zumindest abschnittsweise umgibt, und so zusammen mit dem Funktionsleiter (210) auf diesem Abschnitt eine Doppelleitung bildet.

8. Medizinisches Gerät nach einem der Ansprüche 1 bis 7, **dadurch gekennzeichnet, dass** der Funktionsleiter (210) nicht unterbrochen und einstückig ist, und die Mantelwellensperre um den Funktionsleiter (210) herum oder diesem unmittelbar benachbart angeordnet ist.

9. Medizinisches Gerät nach einem der Ansprüche 1 bis 8, **dadurch gekennzeichnet, dass** der Doppelleitungsabschnitt gewendelt ist.

10. Medizinisches Gerät nach einem der Ansprüche 1 bis 9, **dadurch gekennzeichnet, dass** der Funktionsleiter (210) in dem als Doppelleitung ausgebildeten Abschnitt des medizinischen Gerätes helixartig gewendelt ist.

11. Medizinisches Gerät nach einem der Ansprüche 1 bis 10, **dadurch gekennzeichnet, dass** der Doppelleitungsabschnitt eine Schleife bildet die parallel zu dem Funktionsleiter (210) geführt ist oder bei gewendeltem Funktionsleiter in die Wendellücken mitgewendelt ist.

12. Medizinisches Gerät nach einem der Ansprüche 1 bis 11, **dadurch gekennzeichnet, dass** der Funktionsleiter (210) von einer Isolation umgeben ist, die das Dielektrikum bildet und Zusatzleiter (220) durch Metallisierung des Dielektrikums realisiert ist.

13. Medizinisches Gerät nach einem der Ansprüche 1 bis 11, **dadurch gekennzeichnet, dass** der Außenleiter von einem flexiblen, leitfähigen Polymer oder Liquid Cristal Polymer (LCP) gebildet ist.

14. Medizinisches Gerät nach einem der Ansprüche 1 bis 13, **dadurch gekennzeichnet, dass** der Doppelleitungsabschnitt einen Wellenwiderstand von weniger als 120 Ohm hat.

## Claims

1. A temporarily or permanently implantable medical device comprising at least one elongate electrical function conductor (210) for transmitting therapeutic signals or diagnostic signals or both,
wherein at least one additional conductor (220) is provided, which, together with the function conductor (210), forms, at least in one or more portions, a double line which is separated from the function conductor (210) by a dielectric (230) and which is coupled to the function conductor via a coupling impedance, wherein
the ends of the additional conductor (220) are interconnected via an impedance, wherein the impedance of this connection is dimensioned such that the value of the line wave impedance of the function conductor (210) for frequency ranges far above a frequency range of the therapeutic or diagnostic signals, i.e. in the range of HF fields, is much greater than the value of the line wave impedance of the function conductor (210) in the frequency range of the therapeutic or diagnostic signals, so that currents in a frequency range above the frequency range of the therapeutic or diagnostic signals are damped at least 6 dB more intensely than currents in the 1 kHz range, wherein the one or more portions of the medical device forming a double line forms/form a sheath wave trap, and wherein the coupling impedance is formed by a capacitor or an inductor.

2. The medical device according to claim 1, **characterised in that** the medical device is an electrode line (20) for connection to an implantable heart stimulator (10).

3. The medical device according to either one of claims 1 or 2, **characterised in that** the coupling impedance is formed by a capacitor (240, 350).

4. The medical device according to any one of claims 1 to 3, **characterised in that** the coupling impedance is formed by an inductor.

5. The medical device according to any one of claims 1 to 4, **characterised in that** the portion of the medical device formed as a double line is constructed as a coaxial line, parallel line, or strip line.

6. The medical device according to any one of claims 1 to 5, **characterised in that** the one or more portions of the medical device provided as a double line is/are structurally integrated into the medical device in such a way that the geometric distance between the function conductor (210) and the additional conductor (220) which is connected by the coupling impedance is short in comparison to a wavelength in the double line for a specified maximum working frequency.

7. The medical device according to any one of claims 1 to 6, **characterised in that** the additional conductor (220) is situated proximally of an electrode pole (22, 24, 30, 32) and is near the function conductor (210) at least in one or more portions or surrounds said function conductor at least in one or more portions, and thus together with the function conductor (210) forms a double line at this/these one or more portions.

8. The medical device according to any one of claims 1 to 7, **characterised in that** the function conductor (210) is uninterrupted and is formed in one piece, and the sheath wave trap is situated around or immediately adjacent to the function conductor (210).

9. The medical device according to any one of claims 1 to 8, **characterised in that** the double line portion is coiled.

10. The medical device according to any one of claims 1 to 9, **characterised in that** the function conductor (210) is helically coiled in the one or more portions of the medical device formed as a double line.

11. The medical device according to any one of claims 1 to 10, **characterised in that** the double line section forms a loop which is guided parallel to the function conductor (210), or, for a coiled function conductor, is wound into the coil gaps.

12. The medical device according to any one of claims 1 to 11, **characterised in that** the function conductor (210) is surrounded by insulation that forms the dielectric, and the additional conductor (220) is provided by a metal plating on the dielectric.

13. The medical device according to any one of claims 1 to 11, **characterised in that** the external conductor is formed by a flexible conductive polymer or liquid crystal polymer (LCP).

14. The medical device according to any one of claims 1 to 13, **characterised in that** the double line portion has a wave impedance of less than 120 ohms.

## Revendications

1. Appareil médical implantable de manière temporaire ou permanente avec au moins un conducteur fonctionnel électrique allongé (210) pour la transmission de signaux thérapeutiques ou de signaux de diagnostic, ou des deux,
où au moins un conducteur auxiliaire (220) est prévu, qui forme, ensemble avec le conducteur fonctionnel (210), une ligne double au moins sur des portions, qui est séparé du conducteur fonctionnel (210) par un diélectrique (230) et qui est couplé avec le conducteur fonctionnel par l'intermédiaire d'une impédance de couplage, où les extrémités du conducteur auxiliaire (220) sont reliées ensemble par l'intermédiaire d'une impédance,
où l'impédance de cette connexion est mesurée de telle manière que la valeur de l'impédance des ondes conductrices du conducteur fonctionnel (210) est considérablement plus grande pour des domaines de fréquence bien au-dessus d'un domaine de fréquence des signaux de thérapie ou de diagnostic, de sorte que des flux au-dessus du domaine de fréquence des signaux de thérapie ou de diagnostic sont amortis d'au moins 6 DB supplémentaires que les flux dans le domaine de 1kHz,
où la portion formant une ligne double de l'appareil médical forme un dispositif de blocage d'ondes de surface, et où l'impédance de couplage est formé par une capacité ou une inductance.

2. Appareil médical selon la revendication 1, **caractérisé en ce que** l'appareil médical est une ligne d'électrode (20) pour un raccordement à un stimulateur cardiaque (10) implantable.

3. Appareil médical selon l'une des revendications 1 ou 2, **caractérisé en ce que** l'impédance de couplage est formée par une capacité (240, 350).

4. Appareil médical selon l'une des revendications 1 à 3, **caractérisé en ce que** l'impédance de couplage est formée par une inductance.

5. Appareil médical selon l'une des revendications 1 à 4, **caractérisé en ce que** la portion de l'appareil médical mise sous la forme d'une ligne double est construite sous la forme d'une ligne coaxiale, d'une ligne parallèle ou d'une microbande.

6. Appareil médical selon l'une des revendications 1 à 5, **caractérisé en ce que** la portion de l'appareil médical mise sous la forme d'une ligne double est intégrée de manière constructive dans celui-ci de sorte que la distance géométrique entre le conducteur fonctionnel (210) et le conducteur auxiliaire (220), qui est pontée par l'impédance de couplage, est courte en comparaison avec la longueur d'onde dans la ligne double pour une fréquence de travail élevée prédéfinie.

7. Appareil médical selon l'une des revendications 1 à 6, **caractérisé en ce que** le conducteur auxiliaire (220) est disposé proximal à un pôle d'électrode (22, 24, 30, 32) et est proche du conducteur fonctionnel (210) au moins par portions ou qu'il entoure celui-ci au moins sur des portions, et forme ainsi, ensemble avec le conducteur fonctionnel (210), une ligne double au moins sur cette portion.

8. Appareil médical selon l'une des revendications 1 à 7, **caractérisé en ce que** le conducteur fonctionnel (210) n'est pas interrompu et est en une seule pièce, et le dispositif de blocage d'ondes de surface est disposé autour du conducteur fonctionnel (210) ou au voisinage immédiat de celui-ci.

9. Appareil médical selon l'une des revendications 1 à 8, **caractérisé en ce que** la portion de ligne double est enroulée.

10. Appareil médical selon l'une des revendications 1 à 9, **caractérisé en ce que** le conducteur fonctionnel (210) est enroulé sous forme d'hélice dans la portion de l'appareil médical dans laquelle il est sous la forme d'une ligne double.

11. Appareil médical selon l'une des revendications 1 à 10, **caractérisé en ce que** la portion en ligne double forme une boucle qui est menée parallèlement par rapport au conducteur fonctionnel (210) ou qui est enroulée conjointement dans les creux de l'enroulement lorsque le conducteur fonctionnel est enroulé.

12. Appareil médical selon l'une des revendications 1 à 11, **caractérisé en ce que** le conducteur fonctionnel (210) est entouré d'une isolation qui forme le diélectrique et le conducteur auxiliaire (220) est réalisé par une métallisation du diélectrique.

13. Appareil médical selon l'une des revendications 1 à 11, **caractérisé en ce que** le conducteur externe est formé par un polymère souple conducteur ou un polymère de cristaux liquides (LCP).

14. Appareil médical selon l'une des revendications 1 à 13, **caractérisé en ce que** la portion de ligne double a une impédance d'ondes de moins de 120 Ohm.
